# EUROPEAN PATENT SPECIFICATION

(11) **EP 4 240 246 B1**
(45) Date of publication and mention of the grant of the patent: **07.08.2024**
(21) Application number: 21805886.5
(22) Date of filing: 01.11.2021
(51) Int. Cl.: A61B 8/00

(54) **ROBOTIC SYSTEM FOR PERFORMING AN ULTRASOUND SCAN**
ROBOTERSYSTEM ZUR DURCHFÜHRUNG EINER ULTRASCHALLABTASTUNG
SYSTÈME ROBOTISÉ POUR EFFECTUER UN BALAYAGE À ULTRASONS

(30) Priority: 04.11.2020 DK PA202070729
(43) Date of publication of application: 13.09.2023
(73) Proprietor: Ropca Holding ApS, 5230 Odense M (DK)
(72) Inventor: JØRGENSEN, Johannes Chemnitz Albinus, 5210 Odense NV (DK); SAVARIMUTHU, Thiusius Rajeeth, 5250 Odense SV (DK)
(74) Representative: AWA Denmark A/S
(86) International application number: PCT/EP2021/080273
(87) International publication number: WO 2022/096418

(56) References cited:
- US-A1- 2011 125 022
- US-A1- 2017 252 002
- US-A1- 2020 297 308
- ANDERS CHRISTENSEN: "ROPCA 2019 DEMO - University of Southern Denmark", 27 April 2019 (2019-04-27), XP055886117, Retrieved from the Internet <URL:https://www.youtube.com/watch?v=71Qa5wn97ng>

## Description

### Technical Field

The present invention relates to a robotic system for performing an ultrasound scan on a body part of a patient.

### Background Art

Ultrasound scans are widely employed within the medical world, for example as a diagnostic tool when scanning foetuses, when diagnosing hand or foot injuries, or diseases such as rheumatoid arthritis or gout.

Most ultrasound scan are carried out by a trained medical professional. However, even the most trained ultrasound technicians experience difficulties in performing ultrasound scans in a consistent and efficient manner. Furthermore, only a limited amount of trained medical professionals is out there, thus the wait time for being examined by one can in some cases be very long. The long wait time may lead to a disease or injury worsening before being examined.

To overcome some of the above problems robotic ultrasound systems have been developed. Robotic ultrasound systems may obtain ultrasound scans in an efficient and consistent manner which may lead to lower wait times for patients and improved ultrasound scans.

US 2017/0181725 A1 discloses an ultrasound imaging system including a scanning assembly, a three-dimensional (3D) image acquisition device, and a controller. The scanning assembly is configured to receive a hand or foot and includes a transducer array and an acoustic coupling fluid. The 3D image acquisition device is configured for obtaining a 3D image of the hand or foot. The controller is configured for automatically adjusting direction or orientation of the transducer array with respect to the hand or foot based on the 3D image of the hand or foot.

EP 2 514 366 A1 discloses an exemplary automatic ultrasonic scanning system and a scanning method. The automatic ultrasonic scanning system includes a multi-axis robot arm, an ultrasonic scan head disposed on the multi-axis robot arm, a control circuit for controlling the multi-axis robot arm, three-dimensional image capturing apparatus and a computer. The computer senses a tested object through the three-dimensional image capturing apparatus, creates a three-dimensional shape of the tested object, and plans a three-dimensional scanning path according to the three-dimensional shape. According to the three-dimensional scanning path the computer further controls the multi-axis robot arm to perform a multi-axis motion through the control circuit, so as performs a three-dimensional scan on the tested object through the ultrasonic scan head, and constructs an ultrasonic image according to a reflected ultrasonic signal received by the ultrasonic scan head consequently.

On 27.04.2019, a video was uploaded to YouTube by Anders Christensen (https://www.youtube.com/watch?v=71Qa5wn97ng) . It shows a robotic system for performing an ultrasound scan on a body part, namely a hand, of a patient (see at 0:19), the system comprising:
a. a support surface for supporting the body part (see at 0:19),
b. a glass plate ("glaspladen") (see at 0:19), and
c. a positioning device configured to hold an ultrasound probe and move the ultrasound probe to obtain the ultrasound scan of the body part supported by the support surface (see at 0:19; 0:34),
d. wherein the glass plate is integrated into the support surface and adapted for supporting at least part of the body part to be scanned (see at 0:19).

However, these systems still suffer from drawbacks. The robotic systems are in some cases large and clunky and may take up too much space.

### Summary of the Invention

It is an object of the present invention to provide an improved robotic system, which overcomes or at least alleviates the problems of the prior art.

In a first aspect of the invention, this and further objects are achieved by a robotic system for performing an ultrasound scan on a body part of a patient, the system comprising:
a support surface for supporting the body part,
a display, and
a positioning device configured to hold an ultrasound probe and move the ultrasound probe to obtain the ultrasound scan of the body part supported by the support surface,
wherein the display constitutes at least part of the support surface and is adapted for supporting at least part of the body part to be scanned.

Consequently, a space efficient robotic system is achieved, which does not require additional space for accommodating the display. Having the display constituting at least part of the support surface gives all the possibilities and advantages associated with having a display, some being described in this application, without having the display taking up additional space.

Furthermore, having the display constituting at least part of the support surface further gives possibilities for both medical personnel and a patient to interact with the screen, where in prior art systems the screen is only interacted with by medical personnel.

Having the screen supporting the at least part of the body part assures the at least part of the body part to be scanned will be reachable by the robotic arm and within a scanning volume in which the robotic arm may perform a scan.

The ultrasound scan may be a fully autonomous ultrasound scan carried out without the need for assistance from an operator. The automatic ultrasound scan may be a semi-autonomous ultrasound scan carried out by the positioning device in conjunction with an operator. The operator may provide input to the robotic system regarding movement of the positioning device, e.g. when to initiate the scan, when to stop the scan and/or movement adjustments.

The body of a patient may be any part of the body of a patient, e.g. a foot, a hand, a leg, an arm, a torso, a head, and etc. The robotic system may be capable of performing a full body scan of a patient, or at least perform a scan scanning multiple body parts of a patient.

The support surface may be any surface capable of supporting a body part of a patient. In some embodiments the support surface is a substantially horizontal surface. In some embodiments the support surface extends in a plane with an angle to the horizontal plane. The support surface may be formed as a desk or a table. The support surface may be a planar surface.

In an embodiment the robotic system further comprises secondary support means. The secondary support means may be one or more support protrusions configured to support a body part, such as an arm or a leg. The one or more support protrusions may be formed as one or more braces. The secondary support means may be an additional support surface. The additional support surface may be formed with an indent, formed to at least partly accommodate a body part, such as an arm or a leg. The additional support surface may be arranged abutting the support surface.

The display may be any device capable of displaying one or more colours and patterns. The display may be provided with a receiver, a transmitter and/or a transceiver for receiving and transmitting signals either through a wired or a wireless connection. The display may further comprise a display processing device. The display processing device is a device comprising any circuit and/or device suitably adapted to perform the functions described herein. The display processing device may comprise general purpose or proprietary programmable microprocessors, such as Digital Signal Processors (DSP), Application Specific Integrated Circuits (ASIC), Programmable Logic Arrays (PLA), Field Programmable Gate Arrays (FPGA), special-purpose electronic circuits, etc., or a combination thereof.

The positioning device may be a robotic arm. The positioning device may be any articulated arm configured to hold an ultrasound probe and move the ultrasound probe. The robotic arm may comprise a plurality of joints linked together. The robotic arm may be capable of movement in three dimensions. The articulated arm may comprise a holder for holding the ultrasound probe. The holder may be integrated into an end piece of the robotic arm. The end piece may be the last joint of the robotic arm. Alternatively, the holder may be connectable to the end piece of the robotic arm. The robotic arm may be provided with a receiver, a transmitter and/or a transceiver for receiving and transmitting signals either through a wired or a wireless connection. The robotic arm may further comprise a robotic arm processing device. The robotic arm processing device is a device comprising any circuit and/or device suitably adapted to perform the functions described herein. The robotic arm processing device may comprise general purpose or proprietary programmable microprocessors, such as Digital Signal Processors (DSP), Application Specific Integrated Circuits (ASIC), Programmable Logic Arrays (PLA), Field Programmable Gate Arrays (FPGA), special-purpose electronic circuits, etc., or a combination thereof. The robotic arm may be provided with a sensor. The sensor may be for collecting data regarding movement of the robotic arm, such a sensor could be a gyroscope or an accelerometer. The sensor may be a force sensor configured to detect a force exerted by the robotic arm on a patient undergoing the automatic scan. The sensor may be a force sensor configured to detect a force exerted by the patient on the robotic arm during the scan. The robotic arm processing device may receive data from the sensor and adjust movement of the robotic arm based on the data received. For example, a force threshold may be set for a force exerted by the robotic arm, where if the robotic arm exceeds the force threshold, the robotic arm processing device may stop movement of the robotic arm or move the robotic arm away from the body part being scanned to reduce the force exerted by the robotic arm. Data collected by sensors provided with the robotic arm may be transmitted to a controller configured to control movement of the robotic arm, the controller may then use the received sensor data in controlling movement of the robotic arm.

The positioning device may be a rail system configured to hold the ultrasound probe and move the ultrasound probe in order to obtain the ultrasound scan. The rail system may comprise an ultrasound probe holder connected to a rail of the rail system. The rail system may comprise two rails angled in relation to each, thus allowing movement within a plane defined by the two rails. The two rails may be perpendicular to each other. The rail system may comprise three rails angled in relation to each, thus allowing movement within a 3D space defined by the three rails. The three rails may be perpendicular to each other.

The display may be integrated into the support surface by providing a cut-out, or an indent in the support surface in which the display can be received. The display may be integrated into the support surface by fixedly connecting the display to the support surface. The display may be integrated into the support surface so that a display surface, i.e. the surface of the display displaying patterns and/or colours, is substantially flush with the support surface. The display surface may be angled relative to the horizontal plane when integrated into the support surface. The display surface may be arranged extending within the horizontal plane when integrated into the support surface. In some embodiments the display substantially constitutes the support surface.

The display may be adapted for supporting at least part of the body part to be scanned by providing a planar surface on which at least part of a body part to be scanned may rest. The display may be adapted for supporting the body part to be scanned, e.g. if a hand is to be scanned the whole hand may be supported by the display during scanning. The display may be adapted for supporting at least part the body part to be scanned, e.g. if a hand is to be scanned the fingers or palm of the hand may be supported by the display during scanning. The display may be a planar display. The display may be a curved display. The display may be formed to support a body part.

In some embodiments the display is configured to display an instruction pattern.

By having an instruction pattern displayed a clear reference is given to the robotic arm, which may be used in moving the robotic arm. Furthermore, the instruction pattern may assure that a body part supported by the support surface is within reach of the robotic arm. The instruction outline may alleviate nervousness and stress for a patient as it gives an explicit clear signal.

The instruction pattern may be an instruction outline of a body part to be scanned, e.g. the instruction outline may be of a hand, an arm, a leg, or a foot. Having the instruction outline formed as the body part to be scanned clarifies for a patient where to place their body part to be scanned. Furthermore, having the instruction outline being an instruction outline of a body part may assure the patient places their body part with the correct orientation relative to the robotic arm, which may ease scanning of the body part. Furthermore, it may help the robotic arm in identifying what is currently being scanned, e.g. if the body part being scanned is a pinky or a thumb, as the robot may be aware of the orientation and placement of the body part based on instruction pattern. The instruction pattern may be a cross, a circle, a triangle, a square, a dot, or any other geometric shape. The instruction pattern may indicate where a patient should place their body part to be scanned. Thus, the instruction pattern may indicate a desired placement of a center of the body part to be scanned. The robotic system may comprise a data storage storing a plurality of body outlines and/or instruction patterns. The display may be communicatively connected to the data storage and configured to receive one of the plurality of body outlines and/or instruction patterns from the data storage and display the received body outline or instruction pattern. The display may itself retrieve the body outline or instruction pattern. Alternatively, the body outline or instruction pattern is retrieved via a controller communicatively connected to both the display and the data storage.

In an embodiment, where the instruction pattern is an outline of a body part to be scanned, the outline of the body part displayed may be displayed in correspondence with the patient. For example, if the patient is a child, or an adult, or if the patient is male or female.

Having the instruction pattern chosen dependent on the patient may assure a better match between the outline displayed and the patient body part, consequently, the initial placement of the body part to be scanned may be improved. The display may be configured to receive an input regarding the patient and in response to the input retrieve the outline corresponding to the input received from a data storage storing a plurality of body outlines or instruction patterns. Alternatively, the input may be given to a controller communicatively connectable to the display and the data storage, the controller may then retrieve an outline from the data storage dependent on the input received and transmit the retrieved outline to the display, which may display the received outline. For example, if the input regarding the patient received is that the body part to be scanned is the hand of an adult male, an outline of a generic adult male hand may be retrieved from the data storage and displayed on the display, whereas if the if the input regarding the patient received is that the body part to be scanned is the hand of a female child, an outline of a generic female child hand may be retrieved from the data storage and displayed on the display.

In some embodiments the display is configured to display a message for the patient on which the automatic ultrasound scan is to be performed.

The message may be instructions for the patient on how to place the hand. The message may be regarding progress of automatic scan, e.g. what is being scanned, time left of the scan, or action being performed by the robotic system. The message may be one or more instructions for the patient to move the at least part of the body part supported by the display to correct a placement the at least part of the body part supported by the display. For example, the display may display an outline of a hand, then after the patient have placed their hand against the display, a message may be displayed by the display indicating whether the hand is correctly placed or if the hand should be moved. The message regarding movement of the body part could be a message telling the patient to move their body part up, down, right or left. Alternatively, the message may be an arrow indicating a direction in which the body part should be moved to achieve a correct placement of the body part.

In an embodiment the display is a touch sensitive display.

Consequently, an even more space efficient solution is achieved as added space for a mouse or a keyboard is not needed. The display may then act both as an input device and an output device. For example, information regarding the patient to be scanned may be given directly to the touch sensitive display. For example, the patient may input their gender and age into the touch sensitive display, which may then be transmitted to a controller. The controller may use the received input in generating a movement instruction for the robotic arm, and/or for retrieving pattern and/or outlines to be displayed by the display. In an embodiment the display is provided with an oleophobic coatings or other optical coatings which reduce the visible effects of fingerprint oils. The patient may interact with the touch sensitive display to choose when to initiate the scan or to indicate readiness for the scan, i.e. the hand has been placed and the patient feels ready to be scanned. The operator may interact with the touch sensitive display to display and/or manipulate obtained ultrasound images. The scan or other procedures performed by the robotic system may be initiated and/or stopped by giving an input to the touch sensitive display, e.g. part of the display may act as start and/or a stop button.

In an embodiment the system further comprises a controller for controlling movement of the robotic arm.

The controller comprising any circuit and/or device suitably adapted to perform the functions described herein. The controller may comprise general purpose or proprietary programmable microprocessors, such as Digital Signal Processors (DSP), Application Specific Integrated Circuits (ASIC), Programmable Logic Arrays (PLA), Field Programmable Gate Arrays (FPGA), special-purpose electronic circuits, etc., or a combination thereof. The controller may be provided with a receiver, a transmitter and/or a transceiver for receiving and transmitting signals either through a wired or a wireless connection.

The controller may be communicatively connectable to the screen, to send and/or receive signals from the display. The controller may be communicatively connectable to one or more sensors of the robotic system, to send and/or receive signals from the one or more sensors. The controller may receive input from an operator to initiate a scan, to stop a scan performed by the robotic arm, or to otherwise modify the scan performed by the robotic arm. The controller may be provided with input means allowing an operator to give an input to the controller. The input means may be a touch screen, a keyboard, and/or a mouse. The input means may be configured to receive an input via sound or movement, e.g. voice control, eye movement or gestures. The controller may comprise a data storage. The data storage may store one or more movement instructions for the robotic arm corresponding to one or more different body parts. The data storage may store outlines of body parts. The data storage may store instructions for calibration processes. The data storage may store patient profiles. The controller may be configured to generate movement instructions for moving the robotic arm, based on data received from sensors and/or the display.

In an embodiment the robotic system is configured to:
detect, via the display, an outline of the at least part of the body part supported by the display.

Consequently, the robotic system obtains information regarding the body part, which may be used in moving the robot or for identifying and/or classifying the body part.

The outline of the at least part of the body part may be detected by the display being a touch sensitive display capable of detecting an outline of an object contacting the display. The outline of the at least part of the body part may be detected by the display in conjunction with a sensor. The sensor may be a camera configured to obtain image data of the support surface. The display may be configured for running a pattern and/or colour sequence. The controller may be configured to receive obtained image data and process the image data to see which pixels deviates from the pattern and/or colour sequence displayed by the display. Based on the deviating pixels the controller may determine the outline of the at least part of the body part supported by the display.

The outline of the at least part of the body part detected may be transmitted or calculated by a controller for controlling movement of the robotic arm. The outline detected may be used for controlling the robotic arm, e.g. the controller may generate or adapt movement instructions, based on the detected outline of the body part.

The outline of the at least part of the body part may be saved in a patient profile associated with the patient.

In an embodiment the robotic system is configured to:
detect, via the display, movement and/or the presence of the at least part of the body part supported by the display.

Consequently, the robotic system can adapt to changes in position of the body part. Furthermore, the presence of the body part gives a clear indication whether the scan is ready to be initiated, which further facilitates an autonomous robotic system.

The presence of the body part is to be understood as whether the at least part of the body part is being supported by the display.

Movement and/or the presence of the at least part of the body part supported by the display may be detected by the display being a touch sensitive display capable of detecting movement and/or the presence of an object contacting the display. Movement and/or the presence of the at least part of the body part may be detected by the display in conjunction with a sensor. The sensor may be a camera configured to obtain image data of the support surface and the display may be configured for running a pattern and/or colour sequence. The controller may be configured to receive the obtained image data, and to continuously, or at time intervals, process the image data, to see which pixels deviates from the pattern and/or colour sequence. Based on the deviating pixels over time the controller may determine the movement of the at least part of the body part supported by the display. Alternatively, movement of the at least part of the body part supported by the display may be detected only by the use of a sensor. The sensor may then be a camera configured to obtain image data of the support surface and the at least part of the body part supported by the display, the obtained image data may be transmitted to a controller for analysis, the controller may then determine, based on the analysis of the received image data, whether the at least part of the body part supported by the display has moved.

The presence and/or movement of the at least part of the body part detected may be transmitted or calculated by a controller for controlling movement of the robotic arm. The presence and/or movement detected may be used in controlling the robotic arm, e.g. the controller may adapt movement instructions of the robotic arm in real time in accordance with the presence and/or movement detected.

The presence and/or movement of the at least part of the body part may be used as an emergency stop of the scanning. In an embodiment the robotic system is configured to stop the automatic scan if no presence of the at least part of the body part is detected or if excessive movement of the at least part of the body part is detected. Excessive movement may be defined as the at least part of the body part moving more than 1cm, 2cm, 3cm, 4cm or 5cm.

In an embodiment the system further comprises a 2D sensor and/or a 3D sensor configured to obtain data on the body part of the patient supported by the support surface and/or data on the robotic arm with the ultra sound probe.

Consequently, additional data is gathered which may assist in controlling the robotic arm and/or classifying the body part.

The 2D sensor and/or 3D sensor may be a camera, and/or a LIDAR sensor. In some embodiment the robotic system comprises a sensor assembly comprising a plurality of 2D sensors and/or a plurality of 3D sensors. In some embodiments the 3D sensor is composed of at least two 2D sensors. For example, the 3D sensor may consist of two 2D cameras configured to obtain image data of the support surface and a body part supported by the support surface at different angles. The image data obtained by the two cameras may transmitted and processed by the controller to obtain 3D data regarding the body part supported by the support surface.

The 2D sensor and/or 3D sensor may be configured for obtaining depth data of the body part supported by the support surface, which may be used by the robotic system to create a depth map of the body part supported by the support surface. The depth map may be calculated or transmitted to a controller configured to control movement of the robotic arm. The controller may use the received or calculated depth map in controlling movement of the robotic arm.

The 2D sensor and/or 3D sensor may be configured for obtaining data regarding a position and/or orientation of the ultrasound probe. The 2D sensor and/or 3D sensor may be configured for obtaining data regarding a position and/or orientation of the ultrasound probe relative to the body part supported by the support surface. The position and/or orientation of the ultrasound probe may be transmitted to a controller configured to control movement of the robotic arm. The controller may use the received position and/or orientation of the ultrasound probe in controlling movement of the robotic arm.

The data obtained by the 2D sensor and/or 3D sensor may be processed by the controller to classify a body part to be scanned, e.g. classifying whether a left or a right hand is being scanned or is to be scanned.

The 2D sensor and/or 3D may comprise a receiver, a transmitter, or a transceiver for sending and receiving signals. The 2D sensor and/or 3D sensor may be communicatively connectable to a controller of the robotic system. The controller may be configured to generate one or more control signals for controlling movement of the robotic arm, based on a signal received from the 2D sensor and/or 3D sensor. For example, the 2D sensor and/or 3D sensor may obtain depth data of a body part to be scanned for creating a depth map of the body part to be scanned. The depth data may be obtained prior to the scan being initiated or during the scan. The depth data for creating the depth map may be sent to the controller, which in response to receiving the data creates a depth map of the body part to be scanned. The controller may then generate or adjust a movement instruction for scanning the body part in accordance with the depth map. Alternatively, or in combination, the controller may receive one or more signals from the 2D sensor and/or 3D sensor during scanning or prior to scanning of the body part. The signals may indicate a position and/or orientation of the ultrasound probe relative to the body. The controller may then adjust the movement instruction being executed dependent on the signals indicating the position and/or orientation of the ultrasound probe relative to the body part being scanned or to be scanned.

In an embodiment the robotic system is configured to utilize the display in a calibration process.

Consequently, a controlled calibration of the robotic system may be achieved, which allows the robotic system to work with a high degree of precision.

In an embodiment the robotic system is configured to utilize a calibration pattern and/or calibration colour displayed by the display in the calibration process.

The calibration pattern and/or calibration colour may be a stationary image displayed by the display. The calibration pattern and/or calibration colour may be a sequence of images and/or patterns displayed by the display. The calibration pattern and/or calibration colour may be used for displaying one calibration point for use in calibrating the robotic system. The calibration pattern and/or calibration colour may be used for displaying a plurality of calibration points for use in calibrating the robotic system.

The calibration process may be initiated by an operator giving an initiate input to a controller. The calibration process subsequent to the initiate input may be controlled by the controller or by the controller in conjunction with the operator.

In an embodiment the robotic system is configured to move the robotic arm to contact the calibration pattern and/or calibration colour displayed by the display in the calibration process.

The calibration of the robotic arm may be performed by an operator moving the robotic arm to contact one or more calibration points defined by the calibration pattern and/or calibration colour. The operator may specifically move an ultra sound probe held by an end piece of the robotic arm to contact one or more calibration points defined by the calibration pattern and/or calibration colour. When the robotic arm contacts the one or more calibration points the operator may input to the robotic system that the one or more calibration points have been reached.

The calibration of the robotic arm may be performed by a controller for controlling movement of the robotic arm in conjunction with the 2D and/or 3D sensor configured to obtain data on the body part of the patient supported by the support surface and/or data on the robotic arm with the ultra sound probe. The 2D sensor and/or 3D sensor may transmit information to the controller where the robotic arm is relative to one or more calibration points defined by the calibration pattern and/or calibration colour, e.g. where an ultra sound probe held by an end piece of the robotic arm is relative to one or more calibration points defined by the calibration pattern and/or calibration colour. The controller may generate a movement instruction for the robotic arm, depended on the received information from the 2D sensor and/or 3D, towards the one or more calibration points defined by the calibration pattern and/or calibration colour. The 2D and/or 3D sensor may transmit information to the controller when the robotic arm reaches the one or more calibration points defined by the calibration pattern and/or calibration colour, e.g. whether the ultra sound probe held by the end piece of the robotic arm has reached the one or more calibration points defined by the calibration pattern and/or calibration colour.

In an embodiment the robotic system is configured to utilize a calibration pattern and/or calibration colour displayed by the display to calibrate the 2D sensor and/or 3D sensor. Calibrating the 2D sensor and/or 3D sensor allows for the mapping of 3D coordinates in space to 2D image coordinates. The calibration may be carried out by displaying, via the display, a calibration pattern with known parameters for size, shape, and/or position. The 2D sensor and/or 3D sensor may then obtain image data of the calibration pattern and/or calibration colour displayed. The image data may be correlated with the known parameters of the calibration pattern and/or colour. This may be a fully automatic process, wherein a controller communicatively connected to the display and the 2D and/or 3D sensor comprises the known parameters of the calibration pattern and/or colour and is configured to receive image data from the 2D and/or 3D sensor and correlate these. The correlation by the controller may be to to obtain a transformation matrix allowing mapping of 3D coordinates in space to 2D image coordinates. Thus, image data obtained by the 2D and/or 3D sensor may be used to correctly quantify different geometric metrics in the 3D space.

In an embodiment a display surface of the display is configured to support at least part of the body part, wherein the display surface extends within a display plane with an angle to a horizontal plane of 0-75 degrees, preferably 0-60 degrees, even more preferred 0-45 degrees.

Angling the display surface relative to the vertical plane improves the display's capability of supporting the at least part of the body part.

The display surface is to be understood as the surface of the display capable of displaying a pattern and/or a colour.

In an embodiment the robotic system is a movable unit.

Consequently, a convenient logistic solution is provided, which is not fixed to a single place, but may be moved around.

The robotic system may be provided as a trolley or a table with wheels.

It is noted that the invention relates to all possible combinations of features recited in the claims. Other objectives, features, and advantages of the present inventive concept will appear from the following detailed disclosure, from the attached claims as well as from the drawings. A feature described in relation to one of the aspects may also be incorporated in the other aspect, and the advantage of the feature is applicable to all aspects in which it is incorporated.

### Brief Description of Drawings

In the following description embodiments of the invention will be described with reference to the schematic drawings, in which:
Fig. 1 is a block diagram of a robotic system according to an embodiment of the invention;
Fig. 2 is a schematic perspective view of a robotic system according to an embodiment of the invention;
Fig. 3 is a schematic side view of the robotic system of Fig. 2; and
Fig. 4 is a flow diagram of a calibration process for a robotic system according to an embodiment of the invention.

### Detailed description

The present invention will now be described more fully hereinafter with reference to the accompanying drawings, in which embodiments of the invention are shown. This invention may, however, be embodied in many different forms and should not be construed as limited to the embodiments set forth herein; rather, these embodiments are provided for thoroughness and com pleteness.

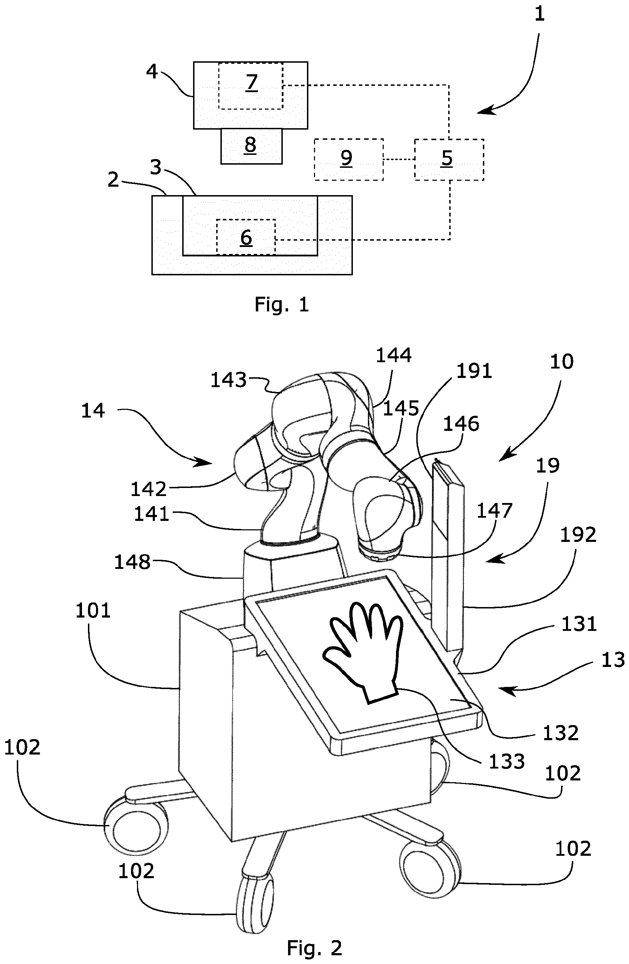

Referring initially to Fig. 1, depicting a block diagram of a robotic system 1 according to an embodiment of the invention. The robotic system 1 being configured for performing an automatic ultrasound scan on a body part of a patient. The robotic system 1 comprises a support surface 2 for supporting the body part to be scanned. The support surface 2 being adapted for receiving a body part and supporting the body part during a scan performed by the robotic system 1. Constituting at least part of the support surface 2 is a display 3. The display 3 is adapted for supporting at least part of the body part to be scanned. Thus, if a patient needs to have a hand scan performed by the robotic system 1, the support surface 2 supports the whole hand and the display 3 constituting at least part of the support surface 2 may support the fingers or the palm of the hand. The display 3 may fully constitute the support surface 2, then the display 3 is adapted for supporting the whole body part to be scanned.

To carry out the automatic scan the robotic system 1 is provided with a robotic arm 4. The robotic arm 4 is configured to hold an ultrasound probe 8 and move the ultrasound probe 8 to obtain the automatic ultrasound scan of the body part supported by the support surface 2.

In the shown embodiment the display 6 is provided with a display processing device 6. The display processing device 6 is communicatively connected to a controller 5. The controller 5 being for controlling movement of the robotic arm 4. The controller 5 is furthermore communicatively connected to the robotic arm processing device 7 provided with the robotic arm 4. The controller 5 is furthermore communicatively connected to a 3D sensor 9. The 3D sensor 9 being configured to obtain data regarding the support surface 2, a body part supported by the support surface 2, and/or the robotic arm 4. The controller 5 may receive signals from the display processing device 6, the robotic arm processing device 7, and/or the 3D sensor 9. The controller 5 may transmit signals to the display processing device 6, the robotic arm processing device 7, and/or the 3D sensor 9. Preferably, the controller 5 receives data from the 3D sensor 9, and/or the display processing device 6, and generates a movement instruction for controlling movement of the robotic arm 4. The movement instruction after being generated is transmitted to the robotic arm processing device 7. After receiving the generated movement instruction from the controller 5, the robotic arm processing device 7 may execute the movement instruction to move the robotic arm 4 in accordance with the received movement instruction.

Referring to Fig. 2, depicting a schematic perspective view of a robotic system 10 according to an embodiment of the invention. In the shown embodiment the robotic arm 14 is provided as an articulated arm 14. The articulated arm consists of a plurality of joints 141, 142, 143, 144, 145, 146. The articulated arm 14 is connected to a system housing 101 via an end joint 141 connected to a base 148 of the robotic arm. The other end joint 146 of the articulated arm 14 is provided with a holder 147. The holder 147 being for holding an ultra sound probe. During scanning the articulated arm 14 moves the holder 147 to obtain a scan of a body part. The joints 141, 142, 143, 144, 145, 146 of the articulated arm 14 may be rotatable independent of each other, thus allowing the articulated arm 14 to perform a wide variety of movements.

In the shown embodiment, the body part is during scanning supported by the display 13. The display 13 constitutes the support surface in the shown embodiment. The display 13 consists of a display housing 131 and a display surface 132. The display surface 132 being for displaying a pattern and/or a colour. The display housing 131 houses the display surface 132. The display surface 132 is provided as a substantially planar surface. The display housing 131 is at one connected to the system housing 101. The display housing 132 may house a display processing device 6 and/or other electronics. The display 13 is a touch sensitive display 13, i.e. an operator or a patient may give input directly to the robotic system 10 via the display surface 132. Input given to the display surface 132 may be transmitted to a controller 5 of the robotic system 10. The controller 5 being for controlling movement of the articulated arm 14. The display 13 in the shown embodiment is configured to display an instruction pattern 133. The instruction pattern 133 is in the shown embodiment is an outline of a body part 133 to be supported by the display 13. The outline of the body part 133 indicates to a patient where to place the body part on the display 13. The outline of the body part 133 displayed is displayed dependent on the patient, i.e. if the patient is an adult female where the right hand is to be scanned, the patient or an operator of the robotic system 10 may give an input to the display 13 or the controller 5 that the patient is an adult female where the right hand is to be scanned, the display 13 may then display the corresponding outline of the body part 133.

Connected to the display housing 131 is a 3D sensor 19. The 3D sensor 19 consists of a sensor stand 192 and a 3D sensor unit 191. The sensor stand 192 is connected to the display housing 131 and extends vertically upwards from the display housing 131. Arranged in the sensor stand 192 is the 3D sensor unit 191. The 3D sensor unit 191 is located vertically above the display 13 in the sensor stand 192. The 3D sensor unit 191 is configured to obtain data on the display 13, a body part supported by the display 13, and/or the articulated arm 14. The data obtained by the 3D sensor unit 191 may be position and/or orientation data of the articulated arm 14 or a body part supported by the display 13. The data obtained by the 3D sensor unit 191 may be 3D data of the body part supported by the display 13, and/or 3D data of the articulated arm 14. The 3D sensor unit may transmit the obtained data to the controller 5 of the robotic system 10. The controller 5 may use the received data for controlling movement of the articulated arm 14.

The robotic system 10 in the shown embodiment is a movable unit 10. The movable unit 10 is achieved by the robotic arm 14 and the display 13 being connected to the system housing 101. The system housing 101 may house the controller 5 and/or other electronics usable by the robotic system 10. The system housing 101 is mounted on a plurality of wheels 102. The plurality of wheels 102 allows for the robotic system 10 to be moved around.

The robotic system 10 may comprise the controller 5 for controlling movement of the articulated arm 14. The controller 10 may be housed in the system housing 101 or be located remotely from the rest of the robotic system 10. The controller 5 may receive inputs from the articulated arm 14, the display 13, and/or the 3D sensor 19. The controller may transmit instructions to the articulated arm 14, the display 13, and/or the 3D sensor 19.

The robotic system 10 is configured to detect, via the display 13, an outline of the body part supported by the display 13. The outline of the body part may be detected by the display 13 being a touch sensitive display 13 capable of detecting an outline of an object contacting the display 13. The outline of the body part may be detected by the display 13 in conjunction with the 3D sensor 19. The 3D sensor 19 may obtain image data of the display 13 and the display 13 may be configured for running a specific pattern and/or colour sequence. The obtained image data may then be transmitted to the controller 5, which is configured to process the image data to see which pixels deviates from the specific pattern and/or colour sequence and determine the outline of the body part supported by the display 13. The outline of the body part detected may be used by the controller 5 for controlling movement of the articulated arm 14.

The robotic system 10 is configured to detect, via the display 13, movement and/or the presence of the body part supported by the display 13. Movement and/or the presence of the body part supported by the display 13 may be detected by the display 13 being a touch sensitive display capable of detecting movement and/or the presence of an object contacting the display. Movement and/or the presence of the body part may be detected by the display 13 in conjunction with the 3D sensor 19. The 3D sensor 19 may be configured to obtain image data of the display 13 and a body part supported by the display 13. The display 13 may be configured for running a pattern and/or colour sequence. Image data obtained by the 3D sensor of the display 13 and a body part supported by the display 13 may be transmitted to the controller 5. The controller 5 may be configured to process the image data, to see over time which pixels deviates from the pattern and/or colour sequence. Based on the overtime deviating pixels, movement of the body part supported by the display may be determined by the controller 5.

The presence and/or movement of the body part detected may be used in controlling the robotic arm, e.g. the controller 5 may adapt movement of the articulated arm 14 in real time in accordance with the presence and/or movement detected. Furthermore, the presence and/or movement of the body part may be used by the controller 5 as an emergency stop. The robotic system may be configured to stop a scan if excessive movement of the body part is detected. Excessive movement may be defined as the body part moving more than 1cm, 2cm, 3cm, 4cm or 5cm.

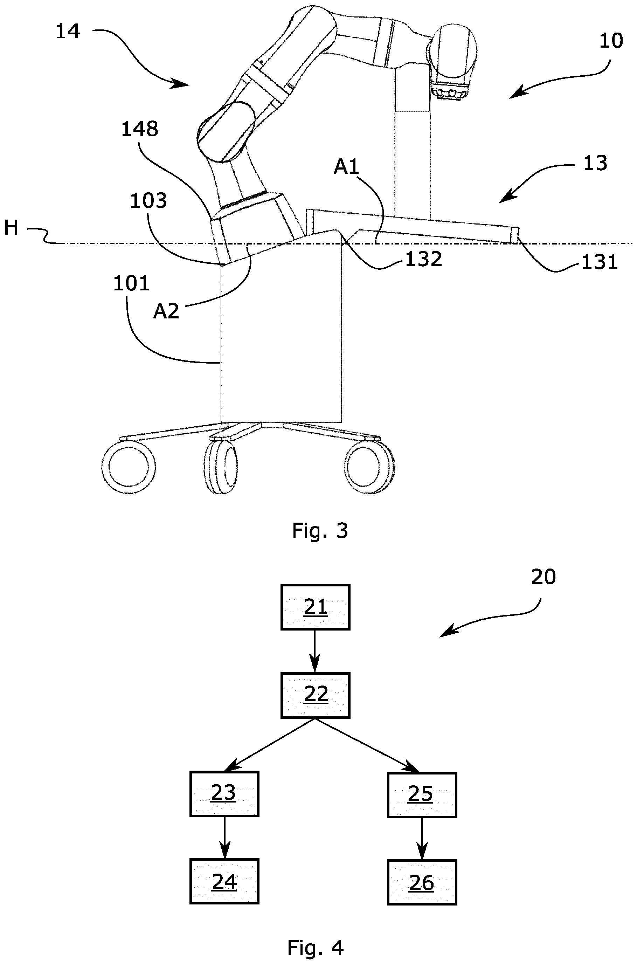

Referring to Fig. 3, depicting a schematic side view of the robotic system 10 of Fig. 2. The display 13 extends with an angle A1 to a horizontal plane H. Preferably, the angle A1 is 0-75 degrees, preferably 0-60 degrees, even more preferred 0-45 degrees. The display housing 131 is at one end provided with a display connection structure 132 facilitating the connection between the display 13 and the system housing 101. The display connection structure 132 is provided as L-shape in display housing 131, wherein the L-shape is configured to be connected to the system housing 101. Furthermore, the articulated arm 14 comprises a base 148. The base 148 being connected to a connection surface 103 of the system housing 101. The connection surface 103 is downwardly angled relative to the horizontal plane H, with a connection angle A2 of 0-60 degrees, preferably 0-45 degrees, even more preferred 0-30 degrees. The connection angle A2 results in the articulated arm 14 being lowered, thus lowering the effective height of the robotic system, and resulting in a more compact robotic system 10.

Referring to Fig. 4, depicting a flow diagram of a calibration process 20 for a robotic system 1, 10 according to an embodiment of the invention. The first step 21 comprises initiating the calibration process 20. The initiation of the calibration process 20 may be done by a controller 5, which is configured to, before a scan of a body part is initiated, to calibrate the robotic system 10. The initiation of the calibration process 20 may be done periodically by the controller when no scan of a body part is being carried out. The initiation of the calibration process 20 may be done in response to an operator inputting to the controller 5 that the calibration process is to be initiated.

The second step 22 comprises displaying by the display 3, 13 a calibration pattern and/or a calibration colour. The display 3, 13 may be configured to display the calibration pattern and/or a calibration colour in response to receiving a signal from the controller 5 that the calibration process 20 has been initiated. The calibration pattern and/or calibration colour may be a stationary image displayed by the display 3, 13. The calibration pattern and/or calibration colour may be a sequence of images and/or patterns displayed by the display 3, 13. The calibration pattern and/or calibration colour may be used for displaying one calibration point for use in calibrating the robotic system. The calibration pattern and/or calibration colour may be used for displaying a plurality of calibration points for use in calibrating the robotic system.

The calibration process 20 may be carried out to calibrate different components of the robotic system 1, 10. If a robotic arm 4, 14 of the robotic system 1, 10 is to be calibrated a third step 23 and a fourth step 24 may be carried out. If a 3D sensor 9, 19 of the robotic system 1, 10 is to be calibrated a fifth step 25 and a sixth step 26 may be carried out instead of the third step 23 and the fourth step 24. Alternatively, both the robotic arm 4, 14 and the 3D sensor 9, 19 may be calibrated in parallel, thus the third step 23 and the fourth step 24 is carried out in parallel with the fifth step 25 and the sixth step 26.

The third step 23 comprises moving the robotic arm 4, 14 to contact the calibration pattern and/or calibration colour displayed by the display 3, 13. The calibration of the robotic arm 4, 14 may be performed by an operator moving the robotic arm 4, 14 to contact one or more calibration points defined by the calibration pattern and/or calibration colour. The operator may specifically move an ultra sound probe 8 held by an end piece 146 of the robotic arm 4, 14 to contact one or more calibration points defined by the calibration pattern and/or calibration colour. Alternatively, the movement of the robotic arm 4, 14 to contact one or more calibration points defined by the calibration pattern and/or calibration colour may be determined by the controller 5. The controller may transmit a movement instruction comprising a determined movement to the robotic arm 4, 14 to move the robotic arm 4, 14 according to the determined movement. The controller 5 may determine the movement in conjunction with the 3D sensor 9, 19 configured to obtain image data of the support surface 2 and image data on the robotic arm 4, 14 with the ultra sound probe 8. The 3D sensor 9, 19 may transmit image data to the controller 5. The controller 5 is configured to determine based on the received image data from the 3D sensor 9, 19, where the robotic arm 4, 14 is relative to the one or more calibration points defined by the calibration pattern and/or calibration colour. The controller may then generate a movement instruction based on the location of the robotic arm 4, 14 relative to the one or more calibration points defined by the calibration pattern and/or calibration colour. The movement instruction comprising the movement needed for the robotic arm 4, 14 to reach the one or more calibration points defined by the calibration pattern and/or calibration colour. The movement instruction is transmitted to the robotic arm 4, 14, which then executes the movement. The movement instruction generated by the controller 5 may be a movement instruction for moving the end piece 146 of the robotic arm 14 towards the one or more calibration points. The movement instruction generated by the controller 5 may be a movement instruction for moving the holder 147 of the robotic arm 14 towards the one or more calibration points. The movement instruction generated by the controller 5 may be a movement instruction for moving an ultrasound probe 8 held by the robotic arm 14 towards the one or more calibration points.

The fourth step 24 comprises verifying the robotic arm 4, 14 has reached the one or more calibration points defined by the calibration pattern and/or calibration colour. The verification may be done by the operator inputting to the robotic system that the robotic arm 4, 14 has reached the one or more calibration points. Alternatively, the controller 5 may verify whether the robotic arm has reached the one or more calibration point by analysing the image data received by the 3D sensor 9, 19.

The fifth step 25 comprises obtaining image data by the 3D sensor 9, 19 and transmitting the obtained image data to the controller 5.

The sixth step 26 comprises correlating, by the controller, the received image data to one or more known parameters of the calibration pattern and/or colour displayed by the display 3, 13. The one or more known parameters may7 be stored in a data storage communicatively connected to the controller 5. The correlation by the controller 5 may be to obtain a transformation matrix allowing mapping of 3D coordinates in space to 2D image coordinates.

Additionally, variations to the disclosed embodiments can be understood and effected by the skilled person in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage.

## Claims

1. A robotic system (1) for performing an ultrasound scan on a body part of a patient, the system comprising:
a support surface (2) for supporting the body part a display (3), and
a positioning device (4) configured to hold an ultrasound probe (8) and move the ultrasound probe to obtain the ultrasound scan of the body part supported by the support surface,
wherein the display is integrated into the support surface and adapted for supporting at least part of the body part to be scanned.

2. A robotic system according to claim 1, wherein the display is configured to display an instruction pattern.

3. A robotic system according to any of the preceding claims, wherein the display is a touch sensitive display.

4. A robotic system according to any of the preceding claims, wherein the system further comprises a controller (5) for controlling movement of the positioning device.

5. A robotic system according to any of the preceding claims, wherein the robotic system is configured to:
detect, via the display, an outline of the at least part of the body part supported by the display.

6. A robotic system according to any of the preceding claims, wherein the robotic system is configured to:
detect, via the display, movement and/or the presence of the at least part of the body part supported by the display.

7. A robotic system according to any of the proceeding claims, wherein
the system further comprises a 2D sensor and/or a 3D sensor (9) configured to obtain data on the body part of the patient supported by the support surface and/or data on the robotic arm with the ultra sound probe.

8. A robotic system according to any of the preceding claims, wherein the robotic system is configured to utilize the display in a calibration process.

9. A robotic system according to any of the preceding claims, wherein a display surface of the display is configured to support at least part of the body part, wherein the display surface extends within a display plane with an angle to a horizontal plane of 0-75 degrees, preferably 0-60 degrees, even more preferred 0-45 degrees.

10. A robotic system according to any of the preceding claims, wherein the robotic system is a movable unit (10).

## Patentansprüche

1. Robotersystem (1) zur Durchführung eines Ultraschallscans an einem Körperteil eines Patienten, wobei das System umfasst:
eine Trägeroberfläche (2) zum Tragen des Körperteils, eine Anzeige (3), und
eine Positioniervorrichtung (4), die ausgestaltet ist, um eine Ultraschallsonde (8) zu halten und die Ultraschallsonde zu bewegen, um den Ultraschallscan des durch die Trägeroberfläche getragenen Körperteils zu erhalten,
wobei die Anzeige in die Trägeroberfläche integriert ist und eingerichtet ist, um mindestens einen Teil des zu scannenden Körperteils zu tragen.

2. Robotersystem nach Anspruch 1, wobei die Anzeige ausgestaltet ist, um eine Anweisungsanleitung anzuzeigen.

3. Robotersystem nach einem der vorhergehenden Ansprüche, wobei die Anzeige eine berührungsempfindliche Anzeige ist.

4. Robotersystem nach einem der vorhergehenden Ansprüche, wobei das System des Weiteren eine Steuerung (5) zum Steuern der Bewegung der Positioniervorrichtung umfasst.

5. Robotersystem nach einem der vorhergehenden Ansprüche, wobei das Robotersystem ausgestaltet ist zum: Detektieren einer Kontur des mindestens einen Teils des durch die Anzeige getragenen Körperteils mittels der Anzeige.

6. Robotersystem nach einem der vorhergehenden Ansprüche, wobei das Robotersystem ausgestaltet ist zum: Detektieren von Bewegung und/oder der Anwesenheit des mindestens einen Teils des durch die Anzeige getragenen Körperteils mittels der Anzeige.

7. Robotersystem nach einem der vorhergehenden Ansprüche, wobei das System des Weiteren einen 2D-Sensor und/oder einen 3D-Sensor (9) umfasst, der/die ausgestaltet ist/sind, um Daten zu dem Körperteil des durch die Trägeroberfläche getragenen Patienten und/oder Daten zu dem Roboterarm mit der Ultraschallsonde zu erhalten.

8. Robotersystem nach einem der vorhergehenden Ansprüche, wobei das Robotersystem ausgestaltet ist, um die Anzeige in einem Kalibrierungsprozess zu nutzen.

9. Robotersystem nach einem der vorhergehenden Ansprüche, wobei eine Anzeigeoberfläche der Anzeige ausgestaltet ist, um mindestens einen Teil des Körperteils zu tragen, wobei sich die Anzeigeoberfläche innerhalb einer Anzeigeebene mit einem Winkel zu einer horizontalen Ebene von 0-75 Grad, vorzugsweise 0-60 Grad, noch bevorzugter 0-45 Grad erstreckt.

10. Robotersystem nach einem der vorhergehenden Ansprüche, wobei das Robotersystem eine bewegliche Einheit (10) ist.

## Revendications

1. Système robotisé (1) pour effectuer un balayage à ultrasons sur une partie corporelle d'un patient, le système comprenant :
une surface de support (2) pour soutenir la partie corporelle,
un dispositif d'affichage (3), et
un dispositif de positionnement (4) configuré pour tenir une sonde à ultrasons (8) et déplacer la sonde à ultrasons pour obtenir le balayage à ultrasons de la partie corporelle soutenue par la surface de support,
le dispositif d'affichage étant intégré à la surface de support et adapté pour supporter au moins une partie de la partie corporelle à balayer.

2. Système robotisé selon la revendication 1, le dispositif d'affichage étant configuré pour afficher un modèle d'instruction.

3. Système robotisé selon l'une quelconque des revendications précédentes, le dispositif d'affichage étant un dispositif d'affichage tactile.

4. Système robotisé selon l'une quelconque des revendications précédentes, le système comprenant en outre un dispositif de commande (5) pour commander le mouvement du dispositif de positionnement.

5. Système robotisé selon l'une quelconque des revendications précédentes, le système robotisé étant configuré pour :
détecter, par l'intermédiaire du dispositif d'affichage, un contour de l'au moins une partie de la partie corporelle supportée par le dispositif d'affichage.

6. Système robotisé selon l'une quelconque des revendications précédentes, le système robotisé étant configuré pour :
détecter, par l'intermédiaire du dispositif d'affichage, le mouvement et/ou la présence de l'au moins une partie de la partie corporelle supportée par le dispositif d'affichage.

7. Système robotisé selon l'une quelconque des revendications précédentes, le système comprenant en outre un capteur 2D et/ou un capteur 3D (9) configuré pour obtenir des données sur la partie corporelle du patient soutenue par la surface de support et/ou des données sur le bras robotisé avec la sonde à ultrasons.

8. Système robotisé selon l'une quelconque des revendications précédentes, le système robotisé étant configuré pour utiliser le dispositif d'affichage dans un processus d'étalonnage.

9. Système robotisé selon l'une quelconque des revendications précédentes, une surface d'affichage du dispositif d'affichage étant configurée pour soutenir au moins une partie de la partie corporelle, la surface d'affichage s'étendant dans un plan d'affichage avec un angle par rapport à un plan horizontal de 0-75 degrés, de préférence de 0-60 degrés, plus préférablement de 0-45 degrés.

10. Système robotisé selon l'une quelconque des revendications précédentes, le système robotisé étant une unité mobile (10).
